# FASCICULE DE BREVET EUROPEEN

(11) **EP 1 242 126 B1**
(45) Date de publication et mention de la délivrance du brevet: **10.09.2003**
(21) Numéro de dépôt: 00973131.6
(22) Date de dépôt: 10.11.2000
(51) Int. Cl.: A61L 9/12

(54) **DISPOSITIF POUR LA DIFFUSION D'AROMES**
VORRICHTUNG FÜR DIE DIFFUSION VON AROMEN
DEVICE FOR DIFFUSING AROMAS

(30) Priorité: 17.12.1999 EP 99125212
(43) Date de publication de la demande: 25.09.2002
(73) Titulaire: FIRMENICH SA, 1211 Genève 8 (CH)
(72) Inventeur: HUGON, Alain, F-01170 Crozet (FR)
(74) Mandataire: Salvaterra-Garcia, Maria de Lurdes, Dr.
(86) Numéro de dépôt international: IB0001635
(87) Numéro de publication internationale: WO01043785

(56) Documents cités:
- WO-A-96/28195
- FR-A- 2 670 568

## Description

### Domaine technique et art antérieur

La présente invention concerne un dispositif pour la diffusion d'arômes ainsi qu'un appareil pour l'appréciation olfactive d'arômes comprenant une pluralité de tels dispositifs. Par arôme, on entend ici toute odeur (senteur, fragrance) émise par un composé pur ou une composition odoriférante ou parfumée sous forme liquide.

On connaît déjà plusieurs types de diffuseurs de senteurs, utilisables tant par les professionnels pour tester une nouvelle fragrance pure ou pour réaliser un nouveau mélange parfumé, que par le public en général, par exemple pour le choix d'un parfum. Ces diffuseurs connus sont basés soit sur la formation d'un brouillard odorant (nébulisation) soit sur l'entraînement d'une senteur au moyen d'air ou d'un gaz neutre à partir d'un support poreux ou d'un polymère dans lequel un liquide odoriférant est absorbé.

Or, ces deux types de diffuseurs présentent des inconvénients et ne donnent ainsi pas satisfaction aux utilisateurs. Dans le cas de la nébulisation, l'inconvénient majeur consiste en la présence dans le fluide testé de micro-gouttelettes du produit liquide aromatique pouvant perturber l'appréciation par l'utilisateur de son odeur réelle. Dans le second type de diffuseurs, le support minéral poreux ou le polymère utilisé comme milieu de stockage du liquide odorant peut provoquer des modifications olfactives et ainsi perturber l'appréciation de l'odeur réelle du produit liquide testé.

En conséquence, le but de la présente invention consiste à fournir un dispositif pour la diffusion d'arôme qui permette une utilisation comparable à celle des diffuseurs connus sans toutefois en présenter les inconvénients, notamment en évitant tant la nébulisation du produit liquide odorant à tester que l'usage d'un support poreux pour le stockage par absorption dudit produit.

Le document FR-A-2 670 568 décrit un dispositif pour la diffusion d'arômes comprenant une grille de diffusion agencée obliquement dans un conduit sur laquelle est projeté le liquide à diffuser.

### Description de l'invention

Le dispositif pour la diffusion d'arôme, objet de la présente invention et visant à atteindre le but précité, présente les caractéristiques définies dans la revendication 1.

Un second objet de cette invention consiste en un appareil pour l'appréciation olfactive d'arômes comprenant plusieurs dispositifs pour la diffusion d'arôme selon l'invention et qui présente les caractéristiques définies dans la revendication 6.

La présente invention sera maintenant décrite plus en détail en référence au dessin annexé.

Le dessin annexé illustre, schématiquement et à titre d'exemple, une forme d'exécution du dispositif de diffusion respectivement de l'appareil pour l'appréciation olfactive d'arôme selon l'invention.

### Brève description des dessins

La figure 1 est une vue en coupe d'une forme d'exécution du dispositif de diffusion selon l'invention.
La figure 2 est une vue en plan d'une réalisation de l'appareil pour l'appréciation olfactive d'arômes selon l'invention.
La figure 3 est une vue en coupe selon la ligne A-B de la figure 2.

### Modes d'exécution de l'invention

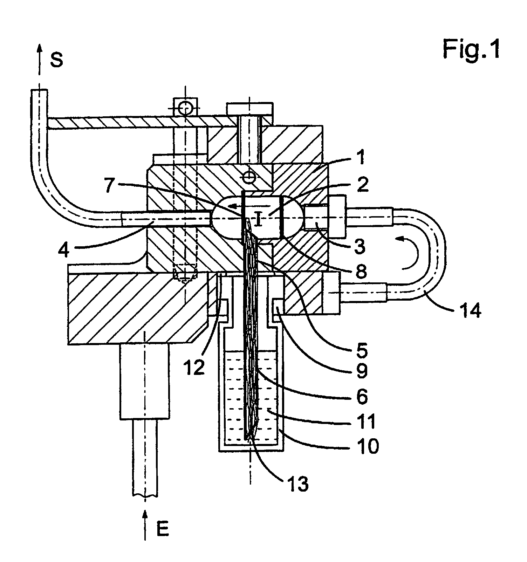

En référence tout d'abord à la figure 1, le dispositif de diffusion selon l'invention illustré comporte un corps principal 1, généralement réalisé en deux parties fixées l'une à l'autre et en un matériau métallique, par exemple en acier inoxydable. présentant une chambre interne 2 et des passages 3, 4 respectivement d'entrée et de sortie d'air, débouchant horizontalement dans ladite chambre 2. Un passage 5 dirigé vers le bas et débouchant à l'extérieur de la face inférieure du corps 1 est également pratiqué à partir de la chambre interne 2. Le corps principal 1, ainsi que les autres parties constitutives, peuvent également être réalisés en un matériau autre que métallique, par exemple en un matériau synthétique, usiné ou moulé, et inerte par rapport aux produits à faire diffuser et aux éventuels solvants les contenant.

De plus, un élément capillaire est disposé dans le passage vers le bas 5 et coopère avec lui, cet élément capillaire étant constitué par exemple d'une aiguille creuse 6. L'extrémité supérieure de cette aiguille creuse 6 est en contact avec une grille de diffusion 7 disposée dans la chambre interne 2 perpendiculairement au flux d'air (flèche I). Le cas échéant, une seconde grille brise-courant 8 peut être disposée dans la chambre interne 2, également perpendiculairement au flux d'air (I), mais en amont par rapport à la grille de diffusion 7.

D'autre part, le corps 1 du dispositif de diffusion présente dans sa partie inférieure et en relation avec le passage vers le bas 5 un logement 9 destiné à recevoir en position de service l'extrémité supérieure d'un flacon 10 servant de réservoir pour le liquide aromatique 11 à tester. De préférence, ce flacon 10 est scellé par exemple avec un diaphragme du type septum 12, celui-ci étant destiné à être percé lors de la mise en place du flacon 10 dans le logement 9 par l'extrémité libre inférieure effilée de l'aiguille creuse 6. En pratique, le flacon 10 est maintenu dans la position de service illustrée par le simple frottement de l'aiguille 6 avec le matériau élastique du septum 12.

En principe, l'aiguille creuse 6 pourrait remplir éventuellement seule son rôle d'élément capillaire pour amener le liquide aromatique 11 du flacon 10 à la grille de diffusion 7, pour autant bien entendu que son diamètre soit approprié. Par contre, en pratique et de préférence, une mèche 13 est disposée à l'intérieur de cette aiguille 6, et par là du passage 5, l'aiguille 6 servant alors de moyen de guidage et de protection pour la mèche 13 qui remplit le rôle d'élément capillaire. Cette mèche 13 est donc en contact d'une part par son extrémité inférieure avec le liquide à diffuser 11 et d'autre part par son extrémité supérieure avec la grille de diffusion 7, sur laquelle elle peut être fixée par exemple au moyen d'un simple point de colle.

De préférence, la mèche 13 est réalisée en un matériau inerte par rapport au produit à diffuser, de telle sorte que, même si cette mèche 13 ne sert que de vecteur audit produit pendant un temps relativement court, elle ne risque pas d'en modifier les caractéristiques chimiques ni surtout olfactives. Des matières appropriées pour la mèche 13 comprennent par exemple le coton, les fibres de cellulose, les fibres de verre ou de roche, etc, le coton étant en principe préféré. De plus, cette mèche 13 peut également ne comporter qu'un nombre très limité de filaments constitutifs (par exemple 2 à 5).

Quant à la grille de diffusion 7 elle-même, elle peut être réalisée avantageusement en une toile d'acier inoxydable ayant des largeurs de maille d'environ 0.15 à 0.4 mm et formée de fils d'environ 0,1 à 0,2 mm de diamètre. Plus particulièrement, on peut utiliser des toiles ayant des largeurs de maille de 0.20, 0.22, 0,25, 0,32 mm, formées respectivement avec des fils de 0,125, 0,16, 0,18, 0,18 mm. Selon des variantes, la grille de diffusion 7 peut être réalisée également en une matière synthétique, par exemple en Téflon®.

En ce qui concerne le fonctionnement du dispositif de diffusion tel que décrit ci-dessus en référence à la figure 1, il consiste à introduire un courant d'air sous pression dans le circuit par l'entrée E, ce circuit d'air passant par une boucle externe 14. puis passant horizontalement selon la flèche 1 à l'intérieur de la chambre interne 2 par l'entremise du passage 3. C'est dans cette chambre 2 que le courant d'air est mis en contact avec le liquide à diffuser, lequel a été amené par capillarité sur la grille de diffusion 7. Le courant d'air peut alors entraîner l'effluve olfactive (espace de tête) avec lui, en ressortant de la chambre 2 par le passage 4, puis en étant dirigé vers l'orifice de sortie 5, où l'utilisateur pourra alors sentir l'odeur de la phase vapeur issue du liquide aromatique à tester, le courant d'air étant exempt de toute micro-gouttelette liquide, ce qui est possible en réglant de manière adéquate la pression de l'air passant à travers le diffuseur.

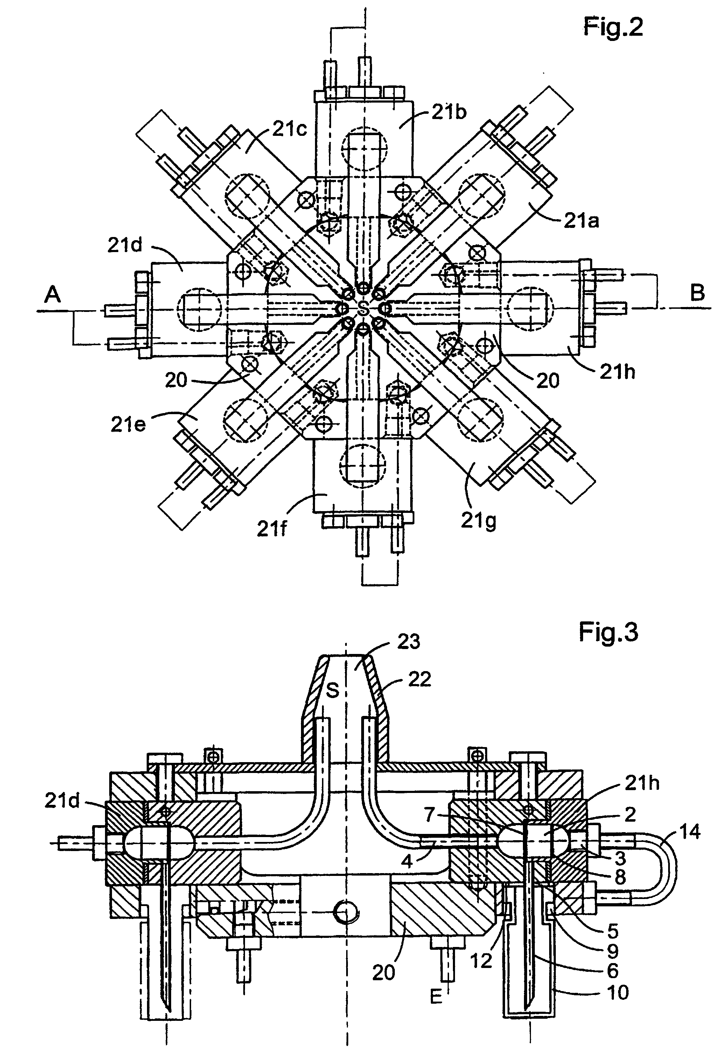

Selon un autre objet de l'invention, plusieurs dispositifs de diffusion, tel que celui décrit ci-dessus, peuvent être disposés radialement en étoile pour former un appareil destiné à l'appréciation olfactive de plusieurs arômes soit séparément soit sous forme de mélanges de plusieurs d'entre eux. Selon la forme d'exécution illustrée à titre d'exemple sur la figure 2, l'appareil comporte un bâti principal 20 circulaire ou polygonal, ici de forme octogonale. Sur chacun des huit côtés de ce bâti 20 est monté un dispositif de diffusion 21a à 21h, par exemple du même type que celui décrit en référence à la figure 1.

Bien entendu, chaque dispositif de diffusion 21a - 21h est asservi à des commandes séparées permettant d'activer chacun d'entre eux soit séparément, un à la fois, soit simultanément à un ou plusieurs autres dispositifs. De préférence, les commandes des diffuseurs sont réunies sur un même panneau, et la source d'air sous pression est commune à tous les dispositifs.

Chacun de ces huit dispositifs 21a à 21h présente une sortie 5 dirigée vers le haut et disposée au centre du bâti 20 de l'appareil, la distance à parcourir par le courant d'air chargé de l'effluve olfactive étant la même pour tous les diffuseurs. De plus, la disposition des huit sorties 4 au centre du bâti 20 permet à l'utilisateur d'activer plusieurs des diffuseurs 2 la - 21 h pour obtenir et apprécier l'odeur d'un mélange de ces effluves, sans se déplacer.

La coupe représentée sur la figure 3 illustre deux dispositifs de diffusion diamétralement opposés, les chiffres de références indiqués sur l'un de ceux-ci correspondant à ceux de la figure 1. De plus, cette figure 3 permet de représenter une variante dans laquelle un capuchon 22 est disposé au-dessus des sorties S des huit dispositifs de diffusion 21a à 21h formant l'appareil illustré sur la figure 2. Ce capuchon 22 présente une ouverture centrale 23 et améliore ainsi les conditions d'utilisation de l'appareil.

Ainsi, grâce à la présente invention, l'utilisateur a à sa disposition, qu'il soit professionnel ou non, un appareil pour l'appréciation olfactive d'un arôme pur ou d'un mélange d'arômes qui est polyvalent, simple à utiliser et facilement transportable de par son faible encombrement et son caractère compact. Par rapport aux diffuseurs connus. le dispositif de diffusion et l'appareil comportant plusieurs de ces dispositifs selon l'invention présentent les avantages suivants :
- stockage en flacon scellé du liquide aromatique permettant une diffusion constante dans le temps et une durée d'utilisation plus grande ;
- facilité d'interchangeabilité des flacons, avantageuse tant pour l'entretien de l'appareil que pour sa polyvalence ;
- absence de micro-gouttelettes liquides dans le courant d'air à sentir par l'utilisateur ;
- risques de modification de caractéristiques olfactives diminués voire supprimés grâce à l'absence de support d'absorption solide ;
- nombreuses possibilités d'utilisation pour les professionnels et pour le public, notamment grâce à la conception radiale en étoile des diffuseurs sur le bâti de l'appareil.

## Revendications

1. Dispositif pour la diffusion d'arôme, **caractérisé par le fait qu'**il comporte un flacon contenant à l'état liquide le produit aromatique à diffuser, ce flacon étant monté de manière amovible au-dessous d'un corps de dispositif, **par le fait que** ledit corps présente une chambre interne communiquant avec ledit flacon en position de service et des passages horizontaux d'entrée respectivement de sortie reliés à un circuit d'air sous pression débouchant dans ladite chambre, **par le fait qu'**une grille de diffusion est disposée à l'intérieur de la chambre interne, perpendiculairement à l'axe des dits passages horizontaux, et **par le fait qu'**un élément capillaire relie le liquide contenu dans le flacon à ladite grille de diffusion.

2. Dispositif selon la revendication 1, **caractérisé par le fait que** l'élément capillaire est une aiguille creuse coopérant avec la grille de diffusion et dont la partie inférieure au moins est disposée en position de service à l'intérieur dudit flacon.

3. Dispositif selon la revendication 2, **caractérisé par le fait qu'**une mèche est disposée à l'intérieur de l'aiguille creuse, l'extrémité supérieure de la mèche étant en contact avec la grille de diffusion et l'extrémité inférieure étant à proximité du fond dudit flacon.

4. Dispositif selon l'une des revendications 1 à 3, **caractérisé par le fait qu'**une grille brise-courant est disposée dans ladite chambre interne, en amont de ladite grille de diffusion et parallèlement à celle-ci.

5. Dispositif selon l'une des revendications 1 à 4, **caractérisé par le fait qu'**il est asservi à des moyens de commande pour régler le passage de l'air sous pression à l'intérieur de la chambre de diffusion.

6. Appareil pour l'appréciation olfactive d'arômes comprenant une pluralité de dispositifs pour la diffusion d'arôme tels que définis dans l'une des revendications 1 à 5. **caractérisé par le fait qu'**il comporte un bâti principal circulaire ou polygonal autour duquel sont montés en étoile lesdits dispositifs pour la diffusion d'arôme, de telle sorte que les tuyaux de sortie que présentent ces dispositifs soient tous concentrés dans la partie centrale dudit bâti.

7. Appareil selon la revendication 6, **caractérisé par le fait qu'**il comporte un capuchon présentant une ouverture centrale dirigée vers le haut et coiffant lesdits tuyaux de sortie.

8. Appareil selon la revendication 6 ou la revendication 7, **caractérisé par le fait que** le bâti est octogonal.

9. Appareil selon l'une des revendications 6 à 8, **caractérisé par le fait que** les moyens de commandes des dispositifs sont assemblés sur un même panneau et que la source d'air sous pression est commune à tous les dispositifs, le tout étant agencé de telle sorte que chaque dispositif puisse être mis en service soit seul soit simultanément à un ou plusieurs autres dispositifs.

## Patentansprüche

1. Vorrichtung zur Duftstoffverteilung, **dadurch gekennzeichnet, dass**
diese ein Fläschchen aufweist, das das zu verteilende wohlriechende Erzeugnis in flüssigem Zustand enthält, wobei dieses Fläschchen abnehmbar unterhalb eines Vorrichtungskörpers montiert ist,
der Körper eine Innenkammer, die mit dem Fläschchen in Arbeitsposition in Verbindung steht, und horizontale Einlass- bzw. Auslasskanäle aufweist, die mit einem in der Kammer mündenden Druckluftkreis verbunden sind,
ein Verteilungsgitter im Inneren der Innenkammer senkrecht zur Achse der Horizontalkanäle angeordnet ist und
ein Kapillarelement die Flüssigkeit, die im Fläschchen enthalten ist, mit dem Verteilungsgitter verbindet.

2. Vorrichtung nach Anspruch 1, **dadurch gekennzeichnet, dass** das Kapillarelement eine Hohlnadel ist, die mit dem Verteilungsgitter zusammenwirkt und deren zumindest innerer Teil in der Betriebsposition im Inneren des Fläschchens angeordnet ist.

3. Vorrichtung nach Anspruch 2, **dadurch gekennzeichnet, dass** sich ein Docht im Inneren der Hohlnadel befindet, wobei der obere Endabschnitt des Dochtes mit dem Verteilungsgitter in Berührung steht und sich der untere Endabschnitt in der Nähe des Bodens vom Fläschchen befindet.

4. Vorrichtung nach einem der Ansprüche 1 bis 3, **dadurch gekennzeichnet, dass** ein Strombrechgitter in der Innenkammer vor dem Verteilungsgitter und parallel zu diesem angeordnet ist.

5. Vorrichtung nach einem der Ansprüche 1 bis 4, **dadurch gekennzeichnet, dass** diese durch eine Steuereinrichtung zum Regeln des Übertritts von Druckluft in das Innere der Verteilungskammer gesteuert wird.

6. Vorrichtung zur Geruchseinschätzung von Duftstoffen mit einer Vielzahl von Vorrichtungen zur Duftstoffverteilung nach einem der Ansprüche 1 bis 5, **dadurch gekennzeichnet, dass** diese ein kreisförmiges oder polygonales Hauptgehäuse aufweist, um das in Sternform die Vorrichtungen zur Duftstoffverteilung herum in einer solchen Weise angebracht sind, dass die Auslassrohre, die diese Vorrichtungen aufweisen, alle im mittleren Teil des Gehäuses konzentriert sind.

7. Vorrichtung nach Anspruch 6, **dadurch gekennzeichnet, dass** diese eine Kappe aufweist, die eine mittlere nach oben gerichtete Öffnung aufweist und die Auslassrohre bedeckt.

8. Vorrichtung nach Anspruch 6 oder 7, **dadurch gekennzeichnet, dass** das Gehäuse achteckig ist.

9. Vorrichtung nach einem der Ansprüche 6 bis 8, **dadurch gekennzeichnet, dass** die Steuereinrichtungen der Vorrichtungen auf einer gleichen Platte verbunden sind und dass die Quelle von Druckluft allen Vorrichtungen gemeinsam ist, wobei das Ganze in der Weise angeordnet ist, dass jede Vorrichtung allein oder gleichzeitig mit einer weiteren Vorrichtung oder mehreren weiteren Vorrichtungen in Betrieb nehmbar ist.

## Claims

1. A device for diffusing aromas, **characterized in that** it comprises a bottle containing the aromatic product to be diffused in the liquid state, said bottle being mounted removably on a device body, **in that** said body has an inner chamber communicating with said bottle in an operating position and have horizontal entrance and respectively exit channels connected to a pressurized air circuit that opens into said chamber, **in that** a diffusion screen is arranged in the inner chamber, perpendicularly to the axis of said horizontal channels, and **in that** a capillary element connects the liquid contained into the bottle to said diffusion screen.

2. A device according to claim 1, **characterized in that** the capillary element is a hollow needle that cooperates with the diffusion screen and at least the lower end of said needle is disposed inside the said bottle in an operating position.

3. A device according to claim 2, **characterized in that** a wick is arranged inside the hollow needle, the upper end of the wick is in contact with the diffusion screen and the lower end is positioned close to the bottom of said bottle.

4. A device according to any one of claims 1 to 3, **characterized in that** a cut-flow screen is arranged in said inner chamber, upstream and in a position parallel to the diffusion screen.

5. A device according to any one of claims 1 to 4, **characterized in that** said device is piloted by operating means for adjusting pressurized air flow in the interior of the diffusion chamber.

6. An apparatus for evaluating aroma odors, comprising a plurality of devices as defined in any one of claims 1 to 5, **characterized in that** it comprises a circular or polygonal main frame around which the plurality of devices are mounted in a star like way, so that the exit channels of the devices are located in the central part of the frame.

7. The apparatus according to claim 6, **characterized in that** it comprises a cover having a central aperture directed upwards and covering the exit channels.

8. An apparatus according to claim 6 or 7, **characterized in that** the frame is octagonal.

9. An apparatus according to any one of claims 6 to 8, **characterized in that** the means for operating the devices are assembled on a same panel and that the source of pressurized air is common for all the devices, the whole being arranged so that each device can be operated individually or simultaneously with one or more other devices.
